(19) Europäisches Patentamt
European Patent Office
Office européen des brevets

(11) Veröffentlichungsnummer: **0 056 486**
**B1**

(12) # EUROPÄISCHE PATENTSCHRIFT

(45) Veröffentlichungstag der Patentschrift: **06.11.85**

(21) Anmeldenummer: **81110718.4**

(22) Anmeldetag: **23.12.81**

(51) Int. Cl.⁴: **C 07 D 233/60**, C 07 D 233/88, C 07 D 249/08, C 07 D 239/42, C 07 D 231/12, C 07 D 261/08, A 61 K 31/41, A 61 K 31/415, A 61 K 31/42, A 61 K 31/505

(54) **Phenylpiperazinyl-propanole von Hetarylphenolen, ihre Herstellung und diese enthaltende therapeutische Mittel.**

(30) Priorität: **19.01.81 DE 3101456**

(43) Veröffentlichungstag der Anmeldung: **28.07.82 Patentblatt 82/30**

(45) Bekanntmachung des Hinweises auf die Patenterteilung: **06.11.85 Patentblatt 85/45**

(84) Benannte Vertragsstaaten: **AT BE CH DE FR GB IT LI LU NL SE**

(56) Entgegenhaltungen:
**AT - B - 317 207**
**DE - A - 2 926 517**
**US - A - 3 577 419**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Franke, Albrecht, Dr., Mandelring 11, D-6706 Wachenheim (DE)**
Erfinder: **Steiner, Gerd, Dr., Oberer Waldweg 1, D-6719 Kirchheim (DE)**
Erfinder: **Thieme, Peter C., Dr., Dr.-Hans-Hoffmann-Strasse 5, D-6706 Wachenheim (DE)**
Erfinder: **Lenke, Dieter, Dr., Kekuleplatz 1, D-6700 Ludwigshafen (DE)**
Erfinder: **Gries, Josef, Dr., Roemerweg 43, D-6706 Wachenheim (DE)**
Erfinder: **Teschendorf, Hans-Juergen, Dr., Rene-Bohn-Strasse 4, D-6700 Ludwigshafen (DE)**

EP 0 056 486 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Phenylpiperazinylpropanole von Hetarylphenolen und ihre physiologisch verträglichen Säureadditionssalze, Verfahren zu ihrer Herstellung und diese Verbindungen enthaltende pharmazeutische Zubereitungen, die vorwiegend blutdrucksenkende, sedative, neuroleptische sowie broncholytische Eigenschaften aufweisen.

In der Patentanmeldung P 3 005 287.0 werden Phenylpiperazinyl-propanole von 1,3,4-Oxadiazolylphenolen vorgeschlagen, die eine blutdrucksenkende Wirkung zeigen. DE-OS 2 926 517 beschreibt Aminopropanole von Hetarylphenolen und deren β-adrenolytische und blutdrucksenkende Wirkung. AT-317 207 beschreibt Phenylpiperazinylpropanole von Hetarylphenolen, deren Hetaryl aus einem Imidazolidinonring besteht, und gibt für sie unter vielem anderen auch eine blutdrucksenkende Wirkung an. Auch die DE-OS 2 824 677 und DE-OS 2 335 432 beschreiben unter anderem eine blutdrucksenkende Wirkung von prinzipiell ähnlichen Verbindungen.

Gegenstand dieser Anmeldung sind unter Abwandlung des heterocyclischen Ringes modifizierte Derivate, die ein unterschiedliches pharmakologisches Wirkungsprofil aufweisen.

Es wurde gefunden, dass Verbindungen der allgemeinen Formel I

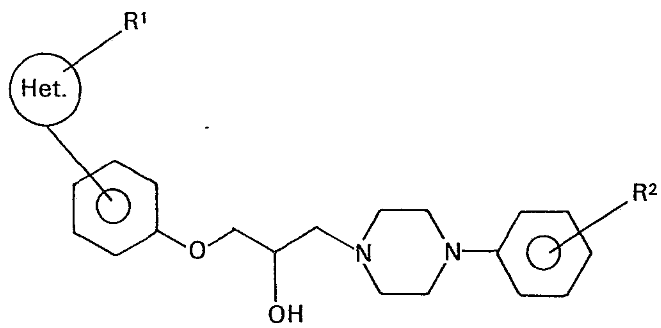

I,

in denen $R^1$ ein Wasserstoffatom, eine Aminogruppe oder einen Alkylrest mit 1 bis 4 C-Atomen, $R^2$ ein Wasserstoffatom, ein Halogenatom, einen Alkylrest mit 1 bis 4 C-Atomen oder einen Alkoxyrest mit 1 bis 3 C-Atomen im Alkyl, wobei $R^2$ als Substituent des Phenylringes ein- oder zweifach vorhanden sein kann, bedeuten und als Heterocyclus Het. ein Pyrimidinrest, ein Triazol-1-yl-, ein Imidazol-1-yl-, ein Pyrazol-3-yl- oder ein Isoxazol-3-yl-rest steht, und ihre Säureadditionssalze wertvolle pharmakologische Eigenschaften aufweisen.

Der Heterocyclus kann sich zu der Ethergruppe in o-, m- oder p-Stellung befinden.

Als Alkylreste $R^1$ mit 1 bis 4 C-Atomen, geradkettig oder verzweigt, seien Methyl, Ethyl, n-Propyl, Isopropyl, n-Butyl und tert.-Butyl genannt.

Der Substituent $R^2$ kann in o-, m- oder p-Stellung zum Piperazinsubstituenten im Phenylring des Phenylpiperazin stehen und beispielsweise folgende Bedeutung haben:

Als Halogenatome kommen Fluor, Chlor, Brom und Jod in Betracht, wobei Fluor und Chlor in p- oder m-Stellung bevorzugt sind. Als niedere Alkoxyreste seien der Methoxy-, Ethoxy-, Propoxy- und Isopropoxyrest genannt, wovon Methoxy- und Ethoxy in o-Stellung bevorzugt sind.

Dementsprechend sind als erfindungsgemässe Verbindungen der Formel I beispielsweise zu nennen:

1-[2-(2-Amino-pyrimidinyl-4)-phenoxy]-3-[4-(4-chlorphenyl)-piperazinyl-1]-propanol-2
1-[2-(2-Amino-pyrimidinyl-4)-phenoxy]-3-[4-(2-methoxyphenyl)-piperazinyl-1]-propanol-2
1-[3-(2-Amino-pyrimidinyl-4)-phenoxy]-3-[4-(3-chlorphenyl)-piperazinyl-1]-propanol-2
1-[3-(2-Amino-pyrimidinyl-4)-phenoxy]-3-[4-(4-chlorphenyl)-piperazin-1]-propanol-2
1-[4-(2-Amino-pyrimidinyl-4)-phenoxy]-3-[4-(3-chlorphenyl)-piperazin-1]-propanol-2
1-[4-(1,2,4-Triazolyl-1)-phenoxy]-3-[4-(4-fluorphenyl-piperazinyl-1]-propanol-2
1-[4-(1,2,4-Triazolyl-1)-phenoxy]-3-[4-(3-methoxyphenyl)-piperazinyl-1]-propanol-2
1-[4-(Imidazolyl-1)-phenoxy]-3-[4-(4-fluorphenyl)-piperazinyl-1]-propanol-2
1-[2-(Pyrazolyl-3)-phenoxy]-3-[4-(4-chlorphenyl)-piperazinyl-1]-propanol-2
1-[2-(Pyrazolyl-3)-phenoxy]-3-[4-(2-methoxyphenyl)-piperazinyl-1]-propanol-2
1-[3-(Pyrazolyl-3)-phenoxy]-3-[4-(2-methoxyphenyl)-piperazinyl-1]-propanol-2
1-[3-(Pyrazolyl-3)-phenoxy]-3-[4-(3-chlorphenyl)-piperazinyl-1]-propanol-2
1-[3-(Pyrazolyl-3)-phenoxy]-3-[4-(4-chlorphenyl)-pipeazinyl-1]-propanol-2
1-[4-(Pyrazolyl-3)-phenoxy]-3-[4-(3-chlorphenyl)-piperazinyl-1]-propanol-2
1-[4-(Pyrazolyl-3)-phenoxy]-3-[4-(4-chlorphenyl)-piperazinyl-1]-propanol-2
1-[2-(Isoxazolyl-3)-phenoxy]-3-[4-(2-methoxyphenyl)-piperazinyl-1]-propanol-2

Bevorzugt sind Verbindungen der allgemeinen Formel I nach Anspruch 1, in denen $R^1$ Wasserstoff oder eine Aminogruppe und $R^2$ ein Fluoratom, Chloratom oder eine Methoxygruppe bedeuten, und ihre physiologisch verträglichen Säureadditionssalze.

Die folgenden Verbindungen sind als besonders bevorzugt und wirksam zu nennen:

1-[2-(2-Amino-pyrimidinyl-4)-phenoxy]-3-[4-(2-methoxyphenyl)-piperazinyl-1]-propanol-2
1-[4-(1,2,4-Triazolyl-1)-phenoxy]-3-[4-(4-fluorphenyl)-piperazinyl-1]-propanol-2
1-[4-(Imidazolyl-1)-phenoxy]-3-[4-(4-fluorphenyl)-piperazinyl-1]-propanol-2
1-[2-(Pyrazolyl-3)-phenoxy]-3-[4-(2-methoxyphenyl)-piperazinyl-1]-propanol-2
1-[3-(Pyrazolyl-3)-phenoxy]-3-[4-(2-methoxyphenyl)-piperazinyl-1]-propanol-2

Die erfindungsgemässen Verbindungen, ausser denen mit einem Pyrimidinrest, werden hergestellt, indem man eine Verbindung der Formel II

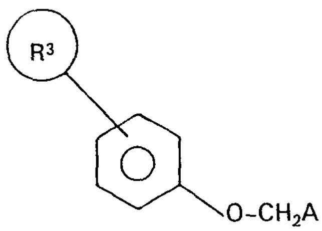

II,

in der $R^3$ einen Triazol-1-yl-, Imidazol-1-yl-, Pyrazol-3-yl- und Isoxazol-3-yl-rest, wobei der heterocyclische Ring durch einen Alkylrest mit 1 bis 4 C-Atomen substituiert sein kann, bedeutet und in der A den Rest

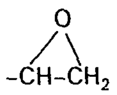

oder

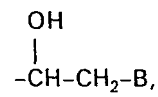

wobei B für eine nukleofuge Abgangsgruppe steht, bedeutet, mit einem Phenylpiperazin der allgemeinen Formel III

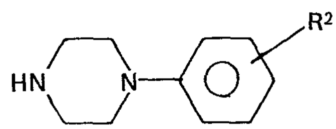

III

in der $R^2$ die für Formel I angegebenen Bedeutungen hat, zweckmässigerweise in einem Lösungsmittel und gegebenenfalls in Gegenwart eines säurebindenden Mittels in an sich bekannter Weise umsetzt und die erhaltene Verbindung der allgemeinen Formel IV

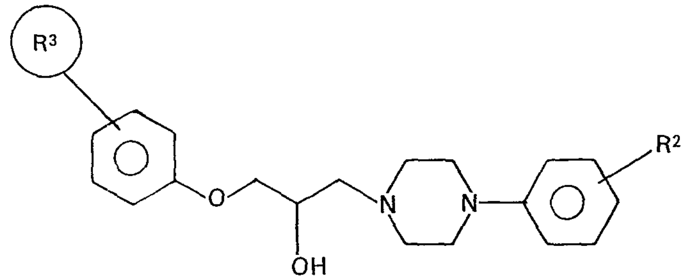

IV,

in der $R^3$ die für Formel II angegebenen Bedeutungen hat, gegebenenfalls in das Säureadditionssalz einer physiologisch verträglichen Säure überführt.

Die Abgangsgruppe B stellt bevorzugt ein Halogenatom, insbesondere Chlor, Brom oder Jod, dar. Weiterhin kommen beispielsweise als nukleofuge Abgangsgruppen aromatische oder aliphatische Sulfonsäurereste in Betracht, wie der p-Toluolsulfonsäure-, der p-Brombenzolsulfonsäure- oder der Methansulfonsäurerest.

Die Umsetzungen werden bei Temperaturen von 10 bis 120°C, d.h. bei Raumtemperaturen oder bei höheren Temperaturen, zweckmässig bei Temperaturen von 50 bis 120°C, durchgeführt. Die Umsetzungen können unter Atmosphärendruck oder in einem geschlossenen Gefäss unter erhöhtem Druck, gegebenenfalls unter Erwärmung auf den angegebenen Temperaturbereich, durchgeführt werden.

Die Ausgangsverbindungen können direkt, d.h. ohne Zugabe eines Verdünnungs- oder Lösungsmittels, umgesetzt werden.

Zweckmässigerweise werden die Umsetzungen jedoch in Gegenwart eines inerten Verdünnungs- oder Lösungsmittels, beispielsweise eines niederen Alkohols mit 1 bis 4 C-Atomen, wie Methanol, Ethanol oder Propanol, vorzugsweise Isopropanol oder Ethanol, eines niederen gesättigten Dialkylethers, Dialkylglykolethers oder cyclischen Ethers, wie Diethylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Octan, eines niederen aliphatischen Ketons, wie Aceton, Methylethylketon oder Methyl-isobutylketon, eines Dialkylformamids, wie Dimethyl- oder Diethylformamid, von Dimethylsulfoxid oder in Gegenwart von Wasser oder in Mischungen der genannten Lösungsmittel, durchgeführt.

Bevorzugte Lösungsmittel bei der Umsetzung eines Epoxids der Formel II mit einem Phenylpiperazin der Formel III sind niedere Alkohole, insbesondere Ethanol, Propanol oder Isopropanol, wobei die Umsetzung bevorzugt bei Temperaturen von 50 bis 120°C und bei Normaldruck durchgeführt wird. Bei der nukleophilen Substitution eines Restes B sind als Lösungsmittel ein niederes aliphatisches Keton, wie Aceton, Diethylketon, Methylisopropylketon oder Methylisobutylketon, ein cyclischer gesättigter Ether, insbesondere Tetrahydrofuran oder Dioxan, oder ein Dialkylformamid, wie Dimethylformamid, und Temperaturen von 90 bis 120°C bevorzugt. Gegebenenfalls wird die Reaktion in Gegenwart einer katalytischen Menge Natrium- oder Kaliumjodid durchgeführt.

Es sei erwähnt, dass als Ausgangsverbindung der Formel II gegebenenfalls auch eine Mischung des Epoxids mit einem Halogenhydrin in Betracht kommen, da bei der technischen Herstellung der Ausgangsverbindungen der Formel II derartige Gemische unter Umständen entstehen können.

Bei einer zweckmässigen Ausführungsform zur nukleophilen Substitution des Restes B durch das verwendete Phenylpiperazinderivat wird die Umsetzung in Gegenwart einer Base als säurebindendes Mittel durchgeführt. Bevorzugte Basen sind Alkalimetallhydroxide, -carbonate, -hydrogencarbonate, -alkoholate oder ein tertiäres organisches Amin wie Pyridin oder ein Trialkylamin, wie Trimethylamin oder Triethylamin. Von den Alkaliverbindungen kommen insbesondere die des Natriums und Kaliums in Betracht. Dabei wird die Base in stöchiometrischer Menge oder in geringem Überschuss verwendet. Gegebenenfalls ist es zweckmässig, das zur Umsetzung verwendete Phenylpiperazinderivat in überschüssiger Menge gleichzeitig als säurebindendes Mittel zu verwenden.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z.B. durch Filtration oder Abdestillieren des Verdünnungs- oder Lösungsmittels aus dem Reaktionsgemisch. Eine Reinigung der erhaltenen Verbindung erfolgt in üblicher Weise, beispielsweise durch Umkristallisieren aus einem Lösungsmittel, Überführen in eine Säureadditionsverbindung oder durch Säulenchromatographie.

Die Ausgangsverbindungen der Formel (II) können durch Alkylierung von Phenolen der allgemeinen Formel V

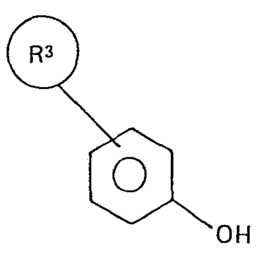

V,

in der $R^3$ die für Formel II angegebenen Bedeutungen hat, mit einem Epihalogenhydrin oder einem $\alpha,\omega$-Dihalogen-2-propanol erhalten werden.

Als Epihalogenhydrine kommen Epichlorhydrin, Epibromhydrin und Epijodhydrin und als $\alpha,\omega$-Dihalogen-2-propanol kommen insbesondere 1,3-Dichlor-2-propanol und 1,3-Dibrom-2-propanol in Betracht.

Die Alkylierungen der Phenol-Derivate der Formel V zur Herstellung der Ausgangsverbindungen der Formel II werden zweckmässigerweise bei Temperaturen von 0 bis 120°C und unter Normaldruck oder in einem geschlossenen Gefäss unter erhöhten Drucken durchgeführt. Die Umsetzungen werden zweckmässigerweise in einem inerten Verdünnungs- oder Lösungsmittel, z.B. einem niederen aliphatischen Keton, wie Aceton, Methylethylketon oder Methylisobutylketon, einem niederen Alkohol mit 1 bis 4 C-Atomen, wie Methanol, Ethanol, Propanol oder Butanol, einem gesättigten aliphatischen oder cyclischen Ether, wie Dialkylether, Tetrahydrofuran oder Dioxan, einem Dialkylformamid, wie Dimethylformamid oder Diethylformamid oder Hexamethylphosphortriamid oder mit überschüssigem Alkylierungsmittel als Verdünnungs- oder Lösungsmittel durchgeführt.

Bevorzugt werden die Umsetzungen in Gegenwart einer Base als säurebindendes Mittel vorgenommen. Geeignete Basen sind Alkalimetallcarbonate, -hydrogencarbonate, -hydroxide, -hydride oder -alkoholate, insbesondere des Natriums und Kaliums, basische Oxide, wie Aluminiumoxid oder Calciumoxid, organische tertiäre Basen, wie Pyridin oder niedere Trialkylamine, wie Trimethyl- oder Triethylamin. Dabei können die Basen im Verhältnis zum eingesetzten Alkylierungsmittel in katalytischer Menge oder in stöchiometrischer Menge bzw. in geringem Überschuss verwendet werden.

Bevorzugt werden die Phenol-Derivate mit Epibromhydrin oder 1,2-Dibrompropanol-2 in einem Lösungsmittelgemisch aus einem Äther und einem polaren aprotischen Lösungsmittel, insbesondere Tetrahydrofuran und Hexamethylphosphortriamid, bei Temperaturen zwischen 0 und 50°C umgesetzt.

Weiterhin sei erwähnt, dass die Ausgangsverbindungen der Formel II durch einfache Säure-Base-Reaktion ineinander umgewandelt werden können. So lässt sich ein 2,3-Epoxypropoxyphenyl-Derivat mit der entsprechenden Halogenwasserstoffsäure in ein 2-Hydroxy-3-halogenpropoxyphenyl-Derivat überführen, wobei als Verdünnungs- oder Lösungsmittel neben an sich üblichen Solventien vorzugsweise aliphatische oder cyclische Ether, wie Diethylether, Tetrahydrofuran oder Dioxan, oder ein niederer Alkohol, wie Methanol, Ethanol oder Propanol, verwendet werden.

Andererseits können die 2-Hydroxy-3-halogenpropoxyphenyl-Derivate mit einer Base, wie einem Alkalimetall-hydroxid, -carbonat, -hydrogencarbonat, -alkoholat oder -hydrid, einem tertiären organischen Amin, wie Pyridin, oder einem tertiären aliphatischen Amin, insbesondere Trimethylamin oder Triethylamin, oder auch Piperidin in 2,3-Epoxypropoxyphenyl-1,3,4-Derivate umgewandelt werden. Diese Reaktionen können bei Raumtemperatur durchgeführt werden oder durch Wärmezufuhr, z.B. durch Erwärmen auf 60 bis 120°C, beschleunigt oder abgeschlossen werden.

Die Reaktion kann unter Atmosphärendruck oder in einem geschlossenen Gefäss unter erhöhtem Druck gegebenenfalls bei gleichzeitigem Erwärmen durchgeführt werden. Die Ausgangsstoffe für diese Umwandlung können zuvor isoliert oder im Reaktionsgemisch erzeugt und ohne weitere Isolierung und Reinigung unmittelbar weiterverarbeitet werden.

Die als Ausgangsverbindungen der Formel V eingesetzten Phenolderivate sind literaturbekannt [DE-OS 2 803 870, DE-OS 2 510 781, J. Org. Chem. 42, 1356 bis 1360 (1977)] oder im Handel erhältlich.

Die erfindungsgemässen Verbindungen der allgemeinen Formel I, in denen

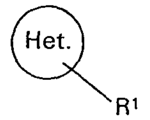

den 2-Amino-pyrimidinyl-4-, den Pyrazolyl-3- oder den Isoxazolyl-3-rest bedeutet, werden hergestellt, indem man ein 1-(3-Dialkylamino-1-oxo-propen)-phenoxy-phenylpiperazinyl-1-propanol-2-Derivat der allgemeinen Formel VI

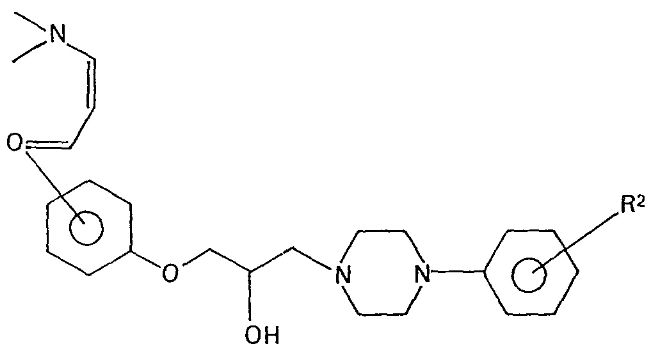

VI

mit Guanidin, Hydroxylamin oder Hydrazinhydrat zweckmässigerweise in Gegenwart eines polaren Lösungsmittels, gegebenenfalls unter Zusatz einer Base, bei Temperaturen zwischen 50 und 120°C in an sich bekannter Weise umsetzt und die erhaltene Verbindung der allgemeinen Formel I mit einem 2-Amino-pyrimidinyl-4-, Isoxazolyl-3- oder Pyrazolyl-3-rest für

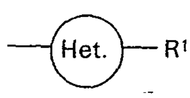

gegebenenfalls in das Säureadditionssalz einer physiologisch verträglichen Säure überführt.

Die Umsetzung wird durch das folgende Formelschema veranschaulicht. Die Verbindungen der Formel VI werden erhalten aus den entsprechenden Carbonylmethylverbindungen, beispielsweise durch Umsetzung mit Dimethylformamidacetal, wie das Formelschema zeigt. Als Dialkylaminogruppe im Oxo-propenrest kommen Dimethyl- und Diethylamino in Betracht.

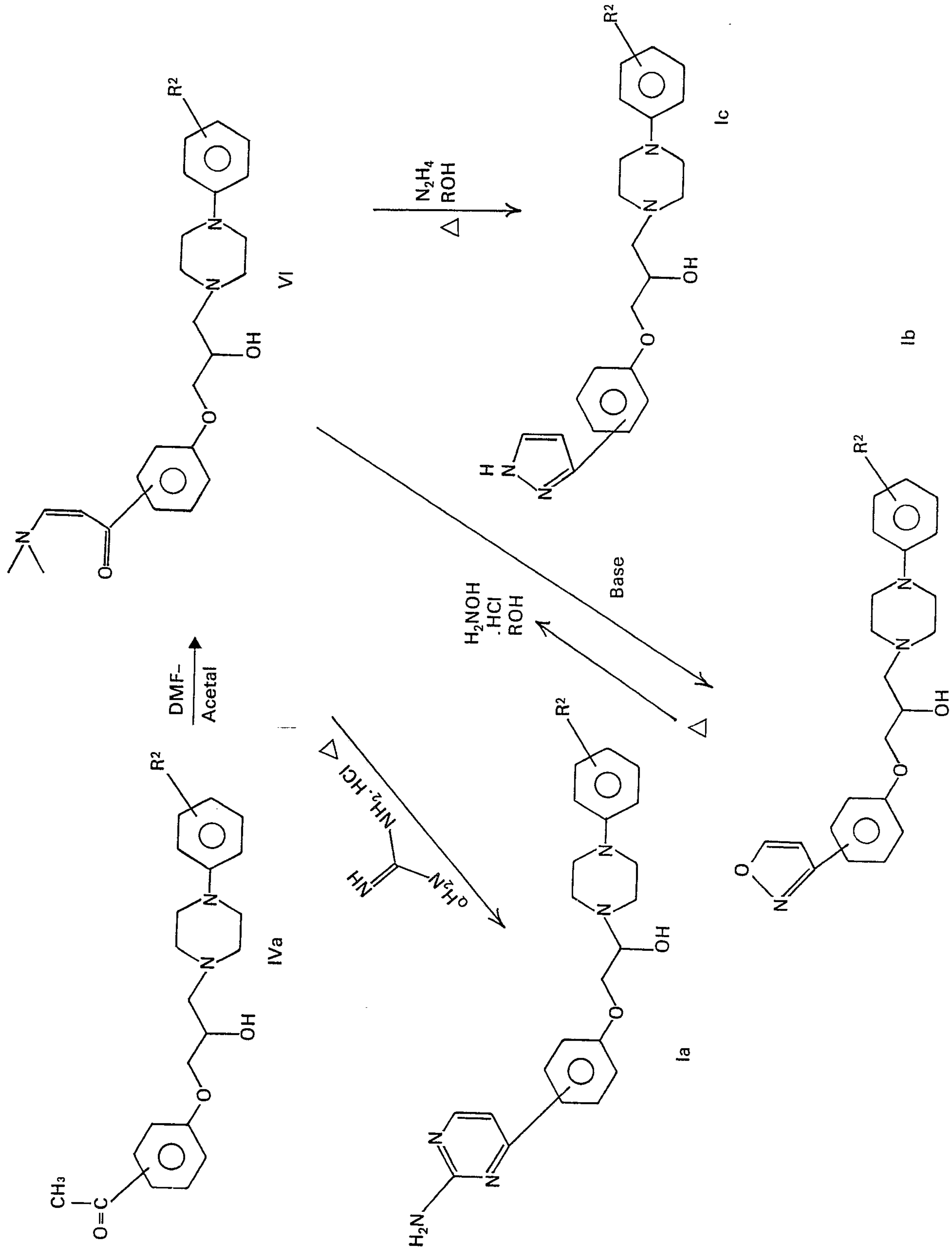


VI Ic Ib IVa Ia

Dabei setzt man das Acetophenon-Derivat IVa mit Dimethylformamidacetal in einem polaren Lösungsmittel wie z.B. einem niederen Alkohol, vorzugsweise Ethanol, bei Temperaturen zwischen 50 und 120°C und Normaldruck zu dem entsprechenden 1-(3-Dialkylamino-1-oxo-propen)-phenoxy-phenylpiperazinyl-1-propanol-2 der allgemeinen Formel VI um. Die Reaktionen sind normalerweise nach 3 bis 8 Tagen beendet.

Anschliessend wird das 1-(3-Alkylamino-1-oxo-propen)-phenoxyphenylpiperazinyl-1-propanol-2-Derivat VI mit Guanidin, Hydroxylamin oder Hydrazinhydrat zu den entsprechenden Endprodukten der allgemeinen Formel Ia, Ib und Ic umgesetzt. Die Umsetzungen werden zweckmässigerweise in Gegenwart eines polaren Lösungsmittels wie einem niederen Alkohol, vorzugsweise Ethanol, gegebenenfalls unter Zusatz einer Base wie einem Alkalialkoholat, vorzugsweise Natriummethylat, bei Temperaturen zwischen 50 und 120°C vorgenommen und sind normalerweise nach 1 bis 10 Tagen beendet.

Die erfindungsgemässen Verbindungen der Formel (I) weisen am Kohlenstoffatom 2 der aliphatischen Seitenkette ein Chiralitätszentrum auf und werden als Racemate erhalten, die durch bekannte Methoden, beispielsweise durch Bildung diastereomerer Salze mit optisch aktiven Hilfssäuren, wie Dibenzoylweinsäure, Campher-10-sulfonsäure, Ditoluylweinsäure oder 3-Bromcampher-8-sulfonsäure, in die optisch aktiven Antipoden getrennt werden können.

Gegebenenfalls werden die erhaltenen erfindungsgemässen Verbindungen in das Säureadditionssalz einer physiologisch verträglichen Säure überführt. Als übliche physiologisch verträgliche anorganische Säuren kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure oder können aus Fortschritte der Arzneimittelforschung, Band 10, Seiten 224 bis 225, Birkhäuser Verlag, Basel und Stuttgart, 1966, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol oder Propanol oder einem niederen Keton wie Aceton, Methylethylketon oder Methylisobutylketon oder einem Ether wie Diethylether, Tetrahydrofuran oder Dioxan, erhalten. Zur besseren Kristallabscheidung können auch Mischungen der genannten Lösungsmittel verwendet werden. Darüber hinaus können pharmazeutisch vertretbare wässrige Lösungen von Säure-Additionsverbindungen der Phenylpiperazinyl-Derivate der allgemeinen Formel (I) durch Auflösen der freien Base der allgemeinen Formel (I) in einer wässrigen Säurelösung hergestellt werden.

Die erfindungsgemässen Verbindungen und ihre physiologisch verträglichen Säureadditionssalze sind als Pharmaka mit blutdrucksenkender, sedativer, neuroleptischer sowie broncholytischer Wirkung zu verwenden.

Hypotensive Wirkung:

Die blutdrucksenkende Wirkung wurde an narkotisierten Ratten (Stamm, Sprague-Dawley, Gewicht: 230 bis 280 g) in Urethan-Narkose (1,78 g/kg intraperitoneal) nachgewiesen. Der Blutdruck wurde in der Arteria carotis gemessen. Die Substanzapplikation erfolgte in eine Vena jugularis (wässrige Lösung, 1 ml/kg) oder intraperitoneal als Suspension in Traganth (10 ml/kg). Als ED 20% wurden aus der linearen Regression zwischen 10 g Dosis (mg/kg) und relativer Blutdrucksenkung ($\Delta$%) die Dosen ermittelt, welche eine 20%ige Blutdrucksenkung bewirken.

Sedative Wirkung:

4 bis 8 Gruppen von jeweils 3 weiblichen NMRI-Mäusen erhalten die Substanz oral appliziert. Die photoelektrische Bestimmung der durch eine neue Umgebung induzierten Orientierungshypermotilität erfolgt 30 min nach der Applikation der Substanzen für eine Dauer von 30 min. Als ED 50% wird die Dosis bestimmt, welche im Vergleich zu Placebo-behandelten Kontrolltieren eine Abnahme der Orientierungshypermotilität um 50% bewirkt.

Kataleptische Wirkung:

Weibliche Sprague-Dawley-Ratten werden 30, 60, 120 und 240 min nach intraperitonealer Substanzapplikation mit den Vorderextremitäten auf eine 8 cm hohe Stange gelegt. Verbleiben die Tiere aufgrund einer Haltungsstarre länger als 15 sec in dieser Position, so gelten sie als kataleptisch. Die Häufigkeit kataleptischer Reaktion pro Dosierung (n/Dosis = 10) wird bestimmt und über Probit-Analyse als ED 50 die Dosis bestimmt, bei der 50% der Tiere kataleptisch sind.

Broncholytische Wirkung:

Männliche oder weibliche Meerschweinchen (Stamm Pribright white, Gewicht 300 bis 500 g) in Urethan-Narkose (1,5 g/kg intraperitoneal) werden künstlich über eine Trachealkanüle beatmet. Durch Injektion von Histamindihydrochlorid (1,0 bis 4,64 µg/kg intravenös) werden Bronchospasmen erzeugt, die durch vorherige intravenöse Applikation der Prüfsubstanzen antagonistisch beeinflusst werden können. Die Registrierung der Spasmen erfolgt nach der Methode von Konzett und Rössler [Naunyn Schmiedeberg's Arch. 195, 71 (1940)] über induktive Druckaufnehmer.

Nach den in Tab. 1 zusammengestellten Ergebnissen zeigen die Verbindungen Beispiel 2 und 3 starke sedativ-neuroleptische Eigenschaften. Bezüglich der Sedation werden 84 bzw. 77%, bezüglich der Katalepsie 43 bzw. 20% der Chlorpromazinwirkung erreicht. Berücksichtigt man die blutdrucksenkende Wirkung – eine Qualität, die für Sedativa/Neuroleptika eine unerwünschte Nebenwirkung darstellt – so sind die Verbindungen

dem Chlorpromazin deutlich überlegen. Der Quotient aus den sedativ wirksamen und den blutdrucksenkenden Dosen liegt um den Faktor 10 bzw. 43 günstiger als beim Chlorpromazin.

Daneben besitzen andere erfindungsgemässe Verbindungen einerseits eine besonders ausgeprägte hypotensive Wirkung und andererseits eine im Verhältnis dazu schwache sedative Wirkung (Tab. 2). So wirken die Verbindungen der Beispiele 10, 5 und 15 in niedrigeren Dosen als Chlorpromazin hypotensiv. Die sedative Nebenwirkung wird erst in rund 400- bis 1300mal höheren Dosen beobachtet (beim Chlorpromazin in 80- resp. 13mal höheren Dosen).

Für die Verbindung Beispiel 1 wurde bei relativ geringer sedativer Wirkung ein starker broncholytischer Effekt am Histamin-Bronchospasmus des Meerschweinchens festgestellt. 0,1 mg/kg intravenös hemmen den Spasmus um durchschnittlich 87%. Die sedative Wirkung (ED 50% = 7,6 mg/kg) erreicht nur 28% des Effektes von Chlorpromazin.

Tabelle 1

| Verbindung Beispiel Nr. | Sedative Wirkung 1) ED 50% | R.W. 4) | Kataleptische W. 2) ED 50% | R.W. 4) | Hypotensive W. 3) ED 20% | R.W. 4) | Q 5) |
|---|---|---|---|---|---|---|---|
| 2 | 2,52 | 0,84 | 10,0 | 0,43 | 0,316 i.v. | 0,08 | 8,0 |
| 3 | 2,75 | 0,77 | 21,5 | 0,2 | 10,0 i.p. | 0,02 | 0,3 |
| Chlor- | 2,11 | 1,0 | 4,3 | 1,0 | 0,026 i.v. | 1,0 | 81,1 |
| promazin | | | | | 0,164 i.p. | 1,0 | 12,9 |

1) Maus, per os
2) Ratte, intraperitoneal
3) Ratte intravenös (i.v.) resp. intraperitoneal (i.p.)
4) Relative Wirksamkeit; Chlorpromazin = 1,00.
5) $Q = \frac{\text{ED 50\% Sedative W.}}{\text{ED 20\% Hypotensive W.}}$

Tabelle 2

| Verbindung Beispiel Nr. | Hypotensive Wirkung ED 20% 1) | R.W. 2) | Sedative Wirkung ED 50% 3) | R.W. 2) | Q 4) |
|---|---|---|---|---|---|
| 10 | 0,0328 i.v. | 0,79 | 14,5 | 0,15 | 442 |
| 5 | 0,0795 i.p. | 2,06 | 32,8 | 0,06 | 413 |
| 15 | 0,0187 i.v. | 1,39 | 25,1 | 0,08 | 1342 |
| Chlor- | 0,0260 i.v. | 1,00 | 2,11 | 1,00 | 81 |
| promazin | 0,164 i.p. | 1,00 | 2,11 | 1,00 | 13 |

1) Dosis, welche den Blutdruck um 20% senkt (Ratte i.v. resp. i.p.)
2) Relative Wirksamkeit; Chlorpromazin = 1,00
3) Dosis, welche die Motilität um 50% reduziert (Maus, per os)
4) $Q = \frac{\text{ED 50\% Sedative W.}}{\text{ED 20\% Hypotensive W.}}$

Gegenstand der vorliegenden Erfindung sind demnach auch therapeutische Mittel oder Zubereitungen, die neben üblichen Träger- und Verdünnungsmitteln eine Verbindung der Formel I oder deren physiologisch verträgliches Säureadditionssalz als Wirkstoff enthalten, sowie die Verwendung der neuen Verbindungen bei der Behandlung der Hypertonie, von psychischen Krankheitszuständen, von Schlafstörungen oder Bronchialerkrankungen.

Die neuen Verbindungen können in den üblichen galenischen Applikationsformen, fest oder flüssig, angewendet werden, wie Tabletten, Kapseln, Pulver, Granulate, Dragees oder Lösungen. Diese werden in üblicher Weise hergestellt. Die Wirkstoffe können dabei mit den gebräuchlichen galenischen Hilfsmitteln, wie Talkum, Gummi arabicum, Saccharose, Lactose, Getreide- oder Maisstärke, Kartoffelmehl, Magnesiumstearat, Alginaten, Gummi tragacanth, Carraghenate, Polyvinylalkohol, Polyvinylpyrrolidon, wässrigen oder nicht wässrigen Trägern, Netzmitteln, Dispergiermitteln, Emulgatoren und/oder Konservierungsmitteln, verarbeitet werden (vgl. L.G. Goodman, A. Gilman, The Pharmacological Basis of Therapeutics). Die so erhaltenen Präparate enthalten den Wirkstoff normalerweise in einer Menge von 0,001 und 99 Gew.%.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Suspensionen oder Depotformen. Es kommen auch parenterale Zubereitungen, wie Injektionslösungen, in Betracht. Weiterhin seien als Zubereitungen

beispielsweise auch Suppositorien genannt.

Entsprechende Tabletten können beispielsweise durch Mischen des Wirkstoffs mit bekannten Hilfsstoffen, beispielsweise inerten Verdünnungsmitteln, wie Dextrose, Zucker, Sorbit, Mannit, Polyvinylpyrrolidon, Calciumcarbonat, Calciumphosphat oder Milchzucker, Sprengmittel wie Maisstärke oder Alginsäure, Bindemittel wie Stärke oder Gelatine, Gleitmittel wie Magnesiumstearat oder Talk und/oder Mittel zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemässen Wirkstoffen können zusätzlich geschmacksverbessernde Mittel, wie Vanillin oder Orangenextrakt, enthalten. Sie können ausserdem Suspendierstoffe, die Natriumcarboxymethylcellulose, oder Konservierungsstoffe, wie p-Hydroxy-benzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt. Geeignete Suppositorien lassen sich beispielsweise durch Vermischen mit dafür vorgesehenen Trägermitteln, wie Neutralfetten oder Polyethylenglykol bzw. deren Derivaten herstellen.

Die Dosierung der erfindungsgemässen Verbindungen hängt vom Alter, Zustand und Gewicht des Patienten sowie von der Applikationsart ab. In der Regel beträgt die tägliche Wirkstoffdosis 5 bis 100, vorzugsweise 10 bis 80 mg.

Die vorliegende Erfindung wird durch die nachfolgenden Ausführungsbeispiele näher erläutert.

Beispiel 1

1-[4-(1,2,4-Triazolyl-1)-phenoxy]-3-[4-(3-methoxyphenyl)-piperazinyl-1]-propanol-2

a) 3,0 g (0,014 Mol) 2,3-Epoxypropoxy-4-(1,2,4-triazolyl-1)-benzol und 2,7 g (0,014 Mol) 1-(2-Methoxy-phenyl-piperazin werden in 60 ml Isopropanol für 10 bis 17 Stunden zum Rückfluss erhitzt. Das Lösungsmittel wird am Rotationsverdampfer abgezogen und der ölige Rückstand in Methylenchlorid aufgenommen, über wasserfreiem Natriumsulfat getrocknet, abfiltriert und eingeengt.

Das als Rückstand anfallende Öl wird in Ether aufgenommen und der entstehende Niederschlag abgesaugt und mit Ether nachgewaschen. Man erhält 2,4 g (42%) Produkt mit Schmp. 145 bis 146°C.

b) Das Vorprodukt 2,3-Epoxypropoxy-4-(1,2,4-triazolyl-1)-benzol wird auf folgende Weise hergestellt: 2,9 g Natriumhydrid als 55%ige Suspension in Paraffinöl (0,067 Mol) werden in 70 ml wasserfreiem Tetrahydrofuran vorgelegt und tropfenweise mit 10,8 g (0,067 Mol) 4-(1,2,4-Triazolyl-1)-phenol gelöst in 50 ml Tetrahydrofuran versetzt. Anschliessend werden 9,2 g (0,067 Mol) Epibromhydrin zugetropft.

Das Reaktionsgemisch lässt man 5 bis 10 Stunden bei Raumtemperatur rühren. Anschliessend giesst man auf 500 ml wässrige Natriumchloridlösung, saugt den ausfallenden Niederschlag ab und wäscht gut mit Wasser nach. Das Rohprodukt wird aus Toluol/Petrolether umkristallisiert. Man isoliert 11,2 g (77%) Produkt mit Schmp. 104 bis 105°C.

Beispiel 2

1-[4-(1,2,4-Triazolyl-1)-phenoxy]-3-[4-(4-fluorphenyl)-piperazinyl-1]-propanol-2

Herstellung analog Beispiel 1a unter Einsatz von 1-(4-Fluorphenyl)-piperazin. Aus dem abgekühlten Reaktionsgemisch kristallisiert beim Stehen über Nacht das Rohprodukt aus, das aus Isopropanol umkristallisiert wird. 74% Ausbeute, Schmp. 149 bis 150°C.

Beispiel 3

1-[4-(Imidazolyl-1)-phenoxy]-3-[4-(4-fluorphenyl)-piperazinyl-1]-propanol-2

a) Herstellung analog Beispiel 1a unter Einsatz von 2,3-Epoxypropoxy-4-(imidazolyl-1)-benzol und 1-(4-Fluorphenyl)-piperazin. Das Rohprodukt wird am besten durch Säulenchromatographie (Kieselgel, Laufmittel Methylenchlorid/Methanol 95/5) und anschliessender Umkristallisation aus Ethanol unter Zusatz von Aktivkohle gereinigt. 51%, Schmp. 144 bis 146°C.

b) Das Vorprodukt 2,3-Epoxypropoxy-4-(imidazolyl-1)-benzol erhält man analog Beispiel 1b unter Einsatz von 4-(Imidazolyl-1)-phenol. Man giesst das Reaktionsgemisch auf Kochsalzlösung und extrahiert mehrmals mit Methylenchlorid. Nach dem üblichen Aufarbeiten der organischen Phase isoliert man das Produkt als Öl, das für die weitere Umsetzung genügend rein ist.

Beispiel 4

1-[2-(2-Amino-pyrimidinyl-4)-phenoxy]-3-[4-(4-chlorphenyl)-piperazinyl-1]-propanol-2

a) 1-(2-Carbonylmethyl-phenoxy)-3-[4-(4-chlorphenyl)-piperazinyl-1]-propanol-2 · HCl

25,0 g (0,13 Mol) 2-(2,3-Epoxypropoxy)-acetophenon in 100 ml Propanol werden mit 26,0 g 1-(4-chlorphenyl)-piperazin versetzt und 4 bis 8 Stunden am Rückfluss gekocht. Nach Abdestillieren des Lösungsmittels nimmt man den Rückstand in wenig Ethanol auf und überführt mit etherischer Salzsäure in das Hydrochlorid, das aus Ethanol/Ether umkristallisiert wird. Man isoliert 33,9 g (61%) Produkt mit Schmp. 186°C.

b) 1-[2-(3-Dimethylamino-1-oxo-propen)-phenoxy]-3-[4-(4-chlorphenyl)-piperazinyl-1]-propanol-2 · HCl

27,0 g (0,064 Mol) 1-(2-Carbonylmethyl-phenoxy)-3-[4-(4-chlorphenyl)-piperazinyl-1]-

propanol-2 · HCl in 250 ml abs. Ethanol werden mit 20 ml Dimethylformamid-dimethylacetal versetzt und 60 Stunden am Rückfluss gekocht. Nach Einengen im Vakuum gibt man zum öligen Rückstand mehrmals Methanol und engt wieder ein. Das so erhaltene Rohprodukt (26,5 g, 86%) ist für die weitere Umsetzung genügend rein.

c) 1-[2-(2-Amino-pyrimidinyl-4)-phenoxy]-3-[4-(4-chlorphenyl)-piperazinyl-1]-propanol-2
10,0 g (0,021 Mol) 1-[2-(3-Dimethylamino-1-oxo-propen)-phenoxy]-3-[4-(4-chlorphenyl)-piperazinyl-1]-propanol-2 · HCl in 250 ml Ethanol werden mit 2,0 g (0,021 Mol) Guanidinhydrochlorid und 0,042 Mol einer 30%igen Natriummethylatlösung versetzt und 12 Stunden am Rückfluss gekocht. Die heisse Lösung wird filtriert und das Filtrat eingeengt. Das Rohprodukt kristallisiert man aus Ethanol um. Man isoliert 3,5 g (38%) Produkt mit Schmp. 159 bis 160°C.

Beispiel 5

1-[2-(2-Amino-pyrimidinyl-4)-phenoxy]-3-[4-(2-methoxyphenyl)-piperazinyl-1]-propanol-2

a) 1-(2-Carbonylmethyl-phenoxy)-3-[4-(2-methoxyphenyl)-piperazinyl-1]-propanol-2
Herstellung analog Beispiel 4a unter Einsatz von 1-(2-Methoxyphenyl)-piperazin. Das Hydrochlorid wurde durch Versetzen mit 2 n Natronlauge und Extraktion mit Methylenchlorid wieder in die freie Base mit Schmp. 116 bis 118°C überführt. 75% Ausbeute.

b) 1-[2-(3-Dimethylamino-1-oxo-propen)-phenoxy]-3-[4-(2-methoxyphenyl)-piperazinyl-1]-propanol-2
Herstellung analog Beispiel 4b unter Einsatz von 1-(2-Carbonylmethyl-phenoxy)-3-[4-(2-methoxyphenyl)-piperazinyl-1]-propanol-2 und Verlängerung des Rückflusskochens auf 8 Tage. 83% Öl.

c) 1-[2-(2-Amino-pyrimidinyl-4)-phenoxy]-3-[4-(2-methoxyphenyl)-piperazinyl-1]-propanol-2
Herstellung analog Beispiel 4c unter Einsatz von 1-[2-(3-Dimethylamino-1-oxo-propen)-phenoxy]-3-[4-(2-methoxyphenyl)-piperazinyl-1]-propanol-2 und Verlängerung des Rückflusskochens auf 8 Tage. 57% Produkt mit Schmp. 147 bis 149°C.

Beispiel 6

1-[3-(2-Amino-pyrimidinyl-4)-phenoxy]-3-[4-(3-chlorphenyl)-piperazinyl-1]-propanol-2

a) 1-(3-Carbonylmethyl-phenoxy)-3-[4-(3-chlorphenyl)-piperazinyl-1]-propanol-2 · HCl
Herstellung analog Beispiel 4a unter Einsatz von 3-(2,3-Epoxypropoxy)-acetophenon und 1-(3-chlorphenyl)-piperazin. 65% mit Schmp. 232 bis 233°C.

b) 1-[3-(3-Dimethylamino-1-oxo-propen)-phenoxy]-3-[4-(3-chlorphenyl)-piperazinyl-1]-propanol-2 · HCl
Herstellung analog Beispiel 4b unter Einsatz von 1-(3-Carbonylmethyl-phenoxy)-3-[4-(3-chlorphenyl)-piperazinyl-1]-propanol-2 · HCl in 85% Ausbeute.

c) 1-[3-(2-Amino-pyrimidinyl-4)-phenoxy]-3-[4-(3-chlorphenyl)-piperazinyl-1]-propanol-2
Herstellung analog Beispiel 4c unter Einsatz von 1-[3-(3-Dimethylamino-1-oxopropen)-phenoxy]-3-[4-(3-chlorphenyl)-piperazinyl-1]-propanol-2 · HCl und Verlängerung des Rückflusskochens auf 2 Tage. 32% Produkt mit Schmp. 140 bis 141°C.

Beispiel 7

1-[3-(2-Amino-pyrimidinyl-4)-phenoxy]-3-[4-(4-chlorphenyl)-piperazinyl-1]-propanol-2

a) 1-(3-Carbonylmethyl-phenoxy)-3-[4-(4-chlorphenyl)-piperazinyl-1]-propanol-2
Herstellung analog Beispiel 4a unter Einsatz von 3-(2,3-Epoxypropoxy)-acetophenon und 1-(4-Chlorphenyl)-piperazin. Nach Abkühlen des Reaktionsgemisches kristallisiert das Produkt aus, das abgesaugt und aus Propanol unter Zusatz von Aktivkohle umkristallisiert wird. 83% Ausbeute.

b) 1-[3-(3-Dimethylamino-1-oxo-propen)-phenoxy]-3-[4-(4-chlorphenyl)-piperazinyl-1]-propanol-2
Herstellung analog Beispiel 4b unter Einsatz von 1-(3-Carbonylmethyl-phenoxy)-3-[4-(4-chlorphenyl)-piperazinyl-1]-propanol-2. Nach Abkühlen des Reaktionsgemisches werden die ausgefallenen Festkörper abgesaugt und aus Ethanol umkristallisiert. 76% mit Schmp. 144 bis 145°C.

c) 1-[3-(2-Amino-pyrimidinyl-4)-phenoxy]-3-[4-(4-chlorphenyl)-piperazinyl-1]-propanol-2
Herstellung analog Beispiel 4c unter Einsatz von 1-[3-(3-Dimethylamino-1-oxo-propen)-phenoxy]-3-[4-(4-chlorphenyl)-piperazinyl-1]-propanol-2 und Verlängerung des Rückflusskochens auf 10 Tage. 49% Produkt mit Schmp. 141 bis 142°C.

Beispiel 8

1-[4-(2-Amino-pyrimidinyl-4)-phenoxy]-3-[4-(3-chlorphenyl)-piperazin-1]-propanol-2

a) 1-(4-Carbonylmethyl-phenoxy)-3-[4-(3-chlorphenyl)-piperazinyl-1]-propanol-2
Herstellung analog Beispiel 4a unter Einsatz von 4-(2,3-Epoxypropoxy)-acetophenon. Nach Abdestillieren des Lösungsmittels wird der Rückstand aus Ethanol umkristallisiert. 89% mit Schmp. 106 bis 107°C.

b) 1-[4-(3-Dimethylamino-1-oxo-propen)-phenoxy]-3-[4-(3-chlorphenyl)-piperazinyl-1]-propanol-2
Herstellung analog Beispiel 4b unter Einsatz von 1-(4-Carbonylmethyl-phenoxy)-3-[4-(3-chlorphenyl)-piperazinyl-1]-propanol-2 und Verlängerung des Rückflusskochens auf 10 Tage. Nach Abkühlen des Reaktionsgemisches saugt man die ausfallenden Festkörper ab und kristallisiert aus Ethanol um. 63% mit Schmp. 130 bis 133°C.

c) 1-[4-(2-Amino-pyrimidinyl-4)-phenoxy]-3-[4-(3-chlorphenyl)-piperazinyl-1]-propanol-2
Herstellung analog Beispiel 4c unter Einsatz von 1-[4-(3-Dimethylamino-1-oxo-propen)-phenoxy]-3-[4-(3-chlorphenyl)-piperazinyl-1]-propanol-2 und Verlängerung des Rückflusskochens auf 4 Tage. 35% Produkt mit Schmp. 197 bis 198°C.

Beispiel 9

1-[2-(Pyrazolyl-3)-phenoxy]-3-[4-(4-chlorphenyl)-piperazinyl-1]-propanol-2

8,0 g (0,017 Mol) 1-[2-(3-Dimethylamino-1-oxo-propen)-phenoxy]-3-[4-(4-chlorphenyl)-piperazinyl-1]-propanol-2 • HCl (Beispiel 4b) in 100 ml Ethanol werden mit 15 ml Hydrazinhydrat versetzt und 3 Tage am Rückfluss gekocht. Nach Abdestillieren des Lösungsmittels kristallisiert man den Rückstand aus Ethanol um. Man isoliert 6,2 g (88%) Produkt mit Schmp. 120 bis 121°C.

Beispiel 10

1-[2-(Pyrazolyl-3)-phenoxy]-3-[4-(2-methoxyphenyl)-piperazinyl-1]-propanol-2 • HCl

Herstellung analog Beispiel 9 unter Einsatz von 1-[2-(3-Dimethylamino-1-oxo-propen)-phenoxy]-3-[4-(2-methoxyphenyl)-piperazinyl-1]-propanol-2 (Beispiel 5b) und Verkürzung der Reaktionszeit auf 4 Stunden. Nach Abdestillieren des Lösungsmittels löst man den Rückstand in Methylenchlorid und wäscht die organische Phase mehrmals mit Wasser aus. Nach dem Trocknen und Einengen überführt man den Rückstand mit etherischer Salzsäure in das Hydrochlorid und kristallisiert aus Ether/Ethanol um. 46% Produkt mit Schmp. 134 bis 135°C.

Beispiel 11

1-[3-(Pyrazolyl-3)-phenoxy]-3-[4-(3-chlorphenyl)-piperazinyl-1]-propanol-2

Herstellung analog Beispiel 9 unter Einsatz von 1-[3-(3-Dimethylamino-1-oxo-propen)-phenoxy]-3-[4-(3-chlorphenyl)-piperazinyl-1]-propanol-2 • HCl (Beispiel 6b). 68% mit Schmp. 154 bis 156°C.

Beispiel 12

1-[3-(Pyrazolyl-3)-phenoxy]-3-[4-(4-chlorphenyl)-piperazinyl-1]-propanol-2

Herstellung analog Beispiel 9 unter Einsatz von 1-[3-(3-Dimethylamino-1-oxo-propen)-phenoxy]-3-[4-(4-chlorphenyl)-piperazinyl-1]-propanol-2 (Beispiel 7b) unter Verlängerung der Reaktionszeit auf 10 Tage. 31% mit Schmp. 152 bis 153°C.

Beispiel 13

1-[4-(Pyrazolyl-3)-phenoxy]-3-[4-(3-chlorphenyl)-piperazinyl-1]-propanol-2

Herstellung analog Beispiel 9 unter Einsatz von 1-[4-(3-Dimethylamino-1-oxo-propen)-phenoxy]-3-[4-(3-chlorphenyl)-piperazinyl-1]-propanol-2 (Beispiel 8b) unter Verlängerung der Reaktionszeit auf 5 Tage. 54% mit Schmp. 189 bis 190°C.

Beispiel 14

1-[4-(Pyrazolyl-3)-phenoxy]-3-[4-(4-chlorphenyl)-piperazinyl-1]-propanol-2

a) 1-(4-Carbonylmethyl-phenoxy)-3-[4-(4-chlorphenyl)-piperazinyl-1]-propanol-2

Herstellung analog Beispiel 4a unter Einsatz von 4-(2,3-Epoxypropoxy)-acetophenon und 1-(4-Chlorphenyl)-piperazin. Das nach Abkühlen des Reaktionsgemisches ausfallende Rohprodukt wird abgesaugt und aus Ethanol/DMF umkristallisiert. 74% mit Schmp. 154°C.

b) 1-[4-(3-Dimethylamino-1-oxo-propen)-phenoxy]-3-[4-(4-chlorphenyl)-piperazinyl-1]-propanol-2

Herstellung analog Beispiel 4b unter Einsatz von 1-(4-Carbonylmethyl-phenoxy)-3-[4-(4-chlorphenyl)-piperazinyl-1]-propanol-2 unter Verlängerung der Reaktionszeit auf 60 Tage. Das nach Abkühlen des Reaktionsgemisches ausfallende Rohprodukt wird abgesaugt und aus Ethanol/DMF umkristallisiert. 47% mit Schmp. 185°C.

c) 1-[4-(Pyrazolyl-3)-phenoxy]-3-[4-(4-chlorphenyl)-piperazinyl-1]-propanol-2

Herstellung analog Beispiel 9 unter Einsatz von 1-[4-(3-Dimethylamino-1-oxo-propen)-phenoxy]-3-[4-(4-chlorphenyl)-piperazinyl-1]-propanol-2 und Verkürzung der Reaktionszeit auf 1 Tag. 69% mit Schmp. 211 bis 212°C.

Beispiel 15

1-[3-(Pyrazolyl-3)-phenoxy]-3-[4-(2-methoxyphenyl)-piperazinyl-1]-propanol-2 • 3 HCl

a) 1-(3-Carbonylmethyl-phenoxy)-3-[4-(2-methoxyphenyl)-piperazinyl-1]-propanol-2 • 2 HCl

Herstellung analog Beispiel 4a unter Einsatz von 4-(2,3-Epoxypropoxy)-acetophenon und 1-(2-Methoxyphenyl)-piperazin. 65% mit Schmp. 206°C (Zersetzung). Die freie Base erhält man durch Extraktion aus alkalischer wässriger Lösung mit Methylenchlorid.

b) 1-[3-(3-Dimethylamino-1-oxo-propen)-phenoxy]-3-[4-(2-methoxyphenyl)-piperazinyl-1]-propanol-2

Herstellung analog Beispiel 4b unter Einsatz von 1-(3-Carbonylmethyl-phenoxy)-3-[4-(2-methoxyphenyl)-piperazinyl-1]-propanol-2 (freie Base). Das nach Abkühlen des Reaktionsgemisches ausfallende Rohprodukt wird abgesaugt und aus Ethanol/DMF umkristallisiert. 34% mit Schmp. 131°C.

c) 1-[3-(Pyrazolyl-3)-phenoxy]-3-[4-(2-methoxyphenyl)-piperazinyl-1]-propanol-2 • 3 HCl

Herstellung analog Beispiel 9 unter Einsatz von 1-[3-(3-Dimethylamino-1-oxo-propen)-phenoxy]-3-[4-(2-methoxyphenyl)-piperazinyl-1]-propanol-2. Nach dem Abdestillieren des Lösungsmittels überführt man den Rückstand mit etherischer Salzsäure in das Hydrochlorid und kristallisiert aus Ether/Ethanol um. 78% mit Schmp. 233 bis 234°C.

Beispiel 16

1-[2-(Isoxazolyl-3)-phenoxy-3-[4-(2-methoxyphenyl)-piperazinyl-1]-propanol-2 • 2 HCl

a) 6,0 g (0,028 Mol) 2,3-Epoxypropoxy-2-(isoxazolyl-3)-benzol und 5,4 g (0,028 Mol) 1-(2-Methoxyphenyl)-piperazin werden in 100 ml n-Propanol 10 Stunden zum Rückfluss erhitzt. Nach Abdestillieren des Lösungsmittels wird der Rückstand mit etherischer Salzsäure in das Hydrochlorid übergeführt. Nach Umkristallisieren aus Methanol/Aceton/Ether isoliert man 7,9 g (59%) Produkt mit Schmp. 210 bis 212°C.

b) Das Vorprodukt 2,3-Epoxypropoxy-2-(isoxazolyl-3)-benzol wird auf folgende Weise hergestellt.

3,0 g Natriumhydrid als 55%ige Suspension in Paraffinöl (0,069 Mol) werden in 100 ml wasserfreiem Tetrahydrofuran vorgelegt und tropfenweise mit 10,0 g (0,062 Mol) 2-(Isoxazolyl-3)-phenol gelöst in 50 ml Tetrahydrofuran und 50 ml Hexamethylphosphorsäuretriamid versetzt. Anschliessend werden 17 g (0,124 Mol) Epibromhydrin zugetropft.

Das Reaktionsgemisch lässt man 5 bis 10 Stunden bei Raumtemperatur rühren. Anschliessend giesst man auf 500 ml wässrige Natriumchloridlösung und extrahiert mehrmals mit Ether. Die organische Phase wird getrocknet und eingeengt. Man erhält das Rohprodukt als Öl, das teilweise durchkristallisiert. Die Kristalle werden abgesaugt und mit wenig Ether nachgewaschen. Man isoliert 6,4 g Produkt (48%) mit Schmp. 64 bis 67°C.

Beispiele für pharmazeutische Zubereitungen:

Beispiele für Tabletten

| | | |
|---|---|---|
| 1. | Ein Wirkstoff der Formel I | 10 mg |
| | Lactose | 200 mg |
| | Methylcellulose | 15 mg |
| | Maisstärke | 50 mg |
| | Talkum | 11 mg |
| | Magnesiumstearat | 4 mg |
| 2. | Ein Wirkstoff der Formel I | 5 mg |
| | Lactose | 178 mg |
| | Avicel | 80 mg |
| | Polywachs 6000 | 20 mg |
| | Magnesiumstearat | 2 mg |
| 3. | Ein Wirkstoff der Formel I | 5 mg |
| | Polyvinylpyrrolidon (mittl. M.G. 25000) | 170 mg |
| | Polyethylenglykol (mittl. M.G. 4000) | 14 mg |
| | Hydroxypropylmethylcellulose | 40 mg |
| | Talkum | 4 mg |
| | Magnesiumstearat | 2 mg |

Zu 3.: Der Wirkstoff wird mit Polyvinylpyrrolidon in 10%iger wässriger Lösung befeuchtet, durch ein Sieb mit der lichten Maschenweite 1,0 mm getrieben und bei 50°C getrocknet. Dieses Granulat wird mit Polyethylenglykol (mittl. M.G. 4000), Hydroxypropylmethylcellulose, Talkum und Magnesiumstearat vermischt und zu Tabletten à 280 mg verpresst.

4. Beispiel für Dragees

| | |
|---|---|
| Ein Wirkstoff der Formel I | 10 mg |
| Lactose | 90 mg |
| Maisstärke | 60 mg |
| Polyvinylpyrrolidon | 6 mg |
| Magnesiumstearat | 1 mg |

Die Mischung der Wirkstoffsubstanz mit Lactose und Maisstärke wird mit einer 8%igen wässrigen Lösung des Polyvinylpyrrolidons durch Sieb 1,5 mm granuliert, bei 50°C getrocknet und nochmals durch Sieb 1,0 mm gerieben. Das so erhaltene Granulat wird mit Magnesiumstearat gemischt und zu Drageekernen verpresst. Die erhaltenen Drageekerne werden in üblicher Weise mit einer Hülle überzogen, die im wesentlichen aus Zucker und Talkum bestehen.

5. Kapselformulierung

| | |
|---|---|
| Ein Wirkstoff der Formel I | 5 mg |
| Magnesiumstearat | 2,0 mg |
| Milchzucker | 19,3 mg |

6. Injektionslösung

| | |
|---|---|
| Ein Wirkstoff der Formel I | 10 mg |
| Natriumchlorid | 9 mg |
| destilliertes Wasser, q.s. auf 1,0 ml | |

**Patentansprüche**

1. Verbindungen der allgemeinen Formel I

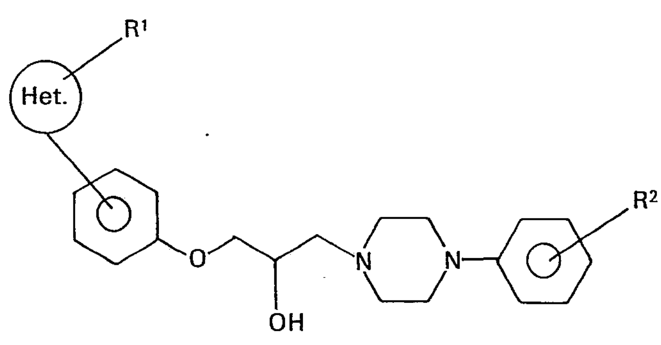

I,

in denen $R^1$ ein Wasserstoffatom, eine Aminogruppe oder einen Alkylrest mit 1 bis 4 C-Atomen, $R^2$ ein Wasserstoffatom, ein Halogenatom, einen Alkylrest mit 1 bis 4 C-Atomen oder einen Alkoxyrest mit 1 bis 3 C-Atomen im Alkyl, wobei $R^2$ als Substituent des Phenylringes ein- oder zweifach vorhanden sein kann, bedeuten und als Heterocyclus Het. ein Pyrimidinrest, ein Triazol-1-yl-, ein Imidazol-1-yl-, ein Pyrazol-3-yl- oder ein Isoxazol-3-yl-rest steht, und ihre pharmakologisch verträglichen Säureadditionssalze.

2. Verbindungen nach Anspruch 1, in denen $R^1$ ein Wasserstoffatom oder eine Aminogruppe, $R^2$ ein Fluoratom, Chloratom oder eine Methoxygruppe bedeuten.

3. 1-[2-(2-Amino-pyrimidinyl-4)-phenoxy]-3-[4-(2-methoxyphenyl)-piperazinyl-1]-propanol-2.

4. 1-[4-(1,2,4-Triazolyl-1)-phenoxy]-3-[4-(4-fluorphenyl)-piperazinyl-1]-propanol-2.

5. 1-[4-(Imidazolyl-1)-phenoxy]-3-[4-(4-fluorphenyl)-piperazinyl-1]-propanol-2.

6. 1-[2-(Pyrazolyl-3)-phenoxy]-3-[4-(2-methoxyphenyl)-piperazinyl-1]-propanol-2.

7. 1-[3-(Pyrazolyl-3)-phenoxy]-3-[4-(2-methoxyphenyl)-piperazinyl-1]-propanol-2.

8. Verfahren zur Herstellung von Verbindungen der allgemeinen Formel I nach Anspruch 1, dadurch gekennzeichnet, dass man eine Verbindung der allgemeinen Formel II

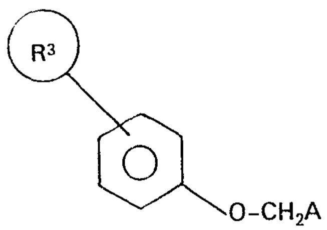

II,

in der $R^3$ einen Triazol-1-yl-, Imidazol-1-yl-, Pyrazol-3-yl- oder Isoxazol-3-yl-rest, wobei der heterocyclische Ring durch einen Alkylrest mit 1 bis 4 C-Atomen substituiert sein kann, bedeutet, und in der A den Rest

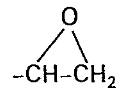

oder

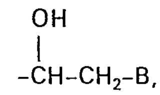

wobei B für eine nukleofuge Abgangsgruppe steht, bedeutet, mit einem Phenylpiperazin der allgemeinen Formel III

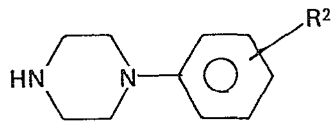

III

in der $R^2$ die für Formel I angegebenen Bedeutungen hat, zweckmässigerweise in einem Lösungsmittel und gegebenenfalls in Gegenwart eines säurebindenden Mittels in an sich bekannter Weise umsetzt und die erhaltene Verbindung gegebenenfalls in das Säureadditionssalz einer physiologisch verträglichen Säure überführt.

9. Verfahren zur Herstellung von Verbindungen der allgemeinen Formel I nach Anspruch 1, dadurch gekennzeichnet, dass man ein 1-(3-Dialkylamino-1-oxo-propen)-phenoxy-phenylpiperazinyl-1]-propanol-2-Derivat der allgemeinen Formel VI

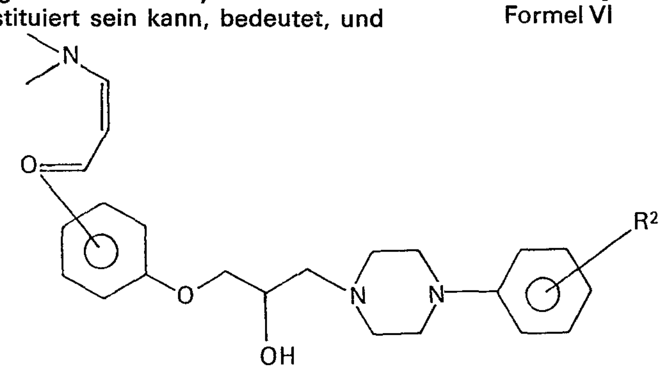

VI

mit Guanidin, Hydroxylamin oder Hydrazinhydrat zweckmässigerweise in Gegenwart eines polaren Lösungsmittels, gegebenenfalls unter Zusatz einer Base, bei Temperaturen zwischen 50 und 120°C in an sich bekannter Weise umsetzt und die erhaltene Verbindung der allgemeinen Formel I mit einem 2-Amino-pyrimidinyl-4-, Isoxazolyl-3- oder Pyrazolyl-3-rest für

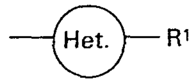

gegebenenfalls in das Säureadditionssalz einer physiologisch verträglichen Säure überführt.

10. Therapeutisches Mittel, gekennzeichnet durch einen Gehalt an einer Verbindung der Formel I oder ihres physiologisch verträglichen Säureadditionssalzes nach Anspruch 1 als Wirkstoff neben üblichen Trägerstoffen und Verdünnungsmitteln.

**Claims**

1. A compound of the general formula I

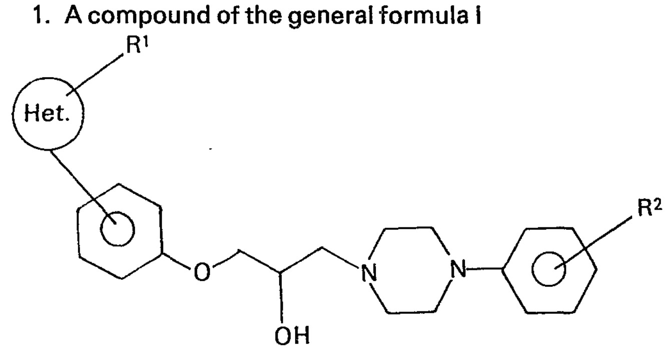

I,

where $R^1$ is hydrogen, amino or alkyl of 1 to 4 carbon atoms, $R^2$ is hydrogen, halogen, alkyl of 1 to 4 carbon atoms or alkoxy where alkyl is of 1 to 3 carbon atoms, the phenyl ring can be monosubstituted or disubstituted by $R^2$, and the heterocyclic structure Het. is pyrimidine, triazol-1-yl, imidazol-1-yl, pyrazol-3-yl or isoxazol-3-yl, and its physiologically tolerated acid addition salts.

2. A compound as claimed in claim 1, where $R^1$ is hydrogen or amino, and $R^2$ is fluorine, chlorine or methoxy.

3. 1-[2-(2-Aminopyrimidin-4-yl)-phenoxy]-3-[4-(2-methoxyphenyl)-piperazin-1-yl]-propan-2-ol.

4. 1-[4-(1,2,4-Triazol-1-yl)-phenoxy]-3-[4-(4-fluorophenyl)-piperazin-1-yl]-propan-2-ol.

5. 1-[4-(Imidazol-1-yl)-phenoxy]-3-[4-(2-fluorophenyl)-piperazin-1-yl]-propan-2-ol.

6. 1-[2-(Pyrazol-3-yl)-phenoxy]-3-[4-(2-methoxyphenyl)-piperazin-1-yl]-propan-2-ol.

7. 1-[3-(Pyrazol-3-yl)-phenoxy]-3-[4-(2-methoxyphenyl)-piperazin-1-yl]-propan-2-ol.

8. A process for the preparation of a compound of the general formula I as claimed in claim 1, wherein a compound of the general formula II

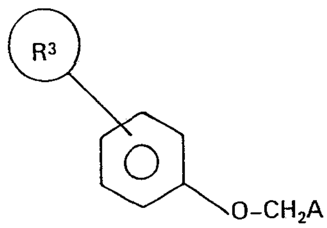

II,

where $R^3$ is triazol-1-yl, imidazol-1-yl, pyrazol-3-yl or isoxazol-3-yl, the heterocyclic ring can be substituted by alkyl of 1 to 4 carbon atoms, and A is

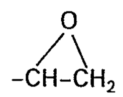

or

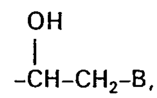

where B is a nucleofugic leaving group, is reacted in a conventional manner with a phenylpiperazine of the general formula III

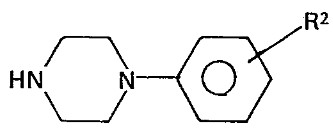

III

where $R^2$ has the meanings given for formula I, advantageously in a solvent and in the presence or absence of an acid acceptor, and the resulting compound is optionally converted into the addition salt of a physiologically tolerated acid.

9. A process for the preparation of a compound of the general formula I as claimed in claim 1, wherein a 1-(3-dialkylamino-1-oxopropenyl)-phenoxyphenyl-piperazin-1-yl-propan-2-ol derivative of the general formula VI

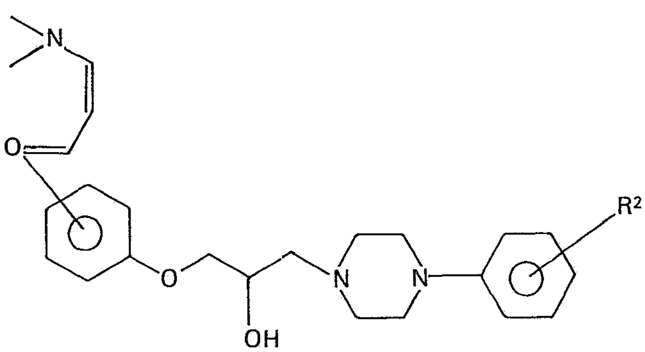

VI

is reacted in a conventional manner with guanidine, hydroxylamine or hydrazine hydrate, advantageously in the presence of a polar solvent, with or without the addition of a base, at from 50 to 120°C, and the resulting compound of the general formula I with 2-aminopyrimidin-4-yl, isoxazol-3-yl or pyrazol-3-yl for

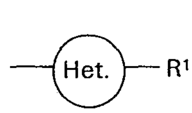

is optionally converted into the addition salt of a physiologically tolerated acid.

10. A therapeutic agent containing a compound of the formula I or a physiologically tolerated acid addition salt thereof as claimed in claim 1, as active ingredient, as well as conventional carriers and diluents.

**Revendications**

1. Composés de la formule générale I

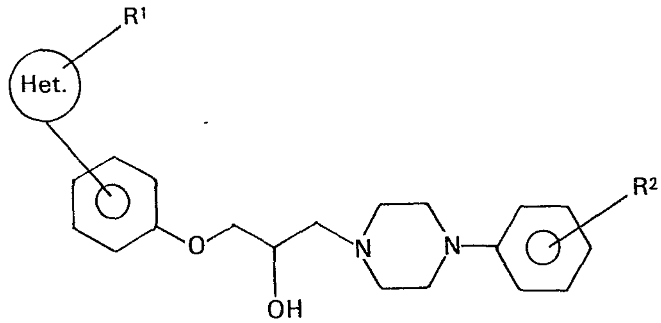

I,

dans laquelle $R^1$ désigne un atome d'hydrogène, un groupe amino ou un radical alkyle en $C_1$ à $C_4$, $R^2$ un atome d'hydrogène ou d'halogène ou un radical alkyle en $C_1$ à $C_4$ ou alcoxy à fraction alkyle en $C_1$ à $C_3$, le noyau phényle pouvant être mono- ou di-substitué par $R^2$, et l'hétérocycle Het est un groupe pyrimidine, triazol-1-yle, imidazol-1-yle, pyrazol-3-yle ou isoxazol-3-yle, ainsi que leurs sels d'addition d'acides physiologiquement acceptables.

2. Composés suivant la revendication 1, pour lesquels $R^1$ désigne un atome d'hydrogène ou un groupe amino et $R^2$ un atome de fluor ou de chlore ou un groupe méthoxy.

3. Le 1-[2-(2-amino-pyrimidinyl-4)-phénoxy]-3-[4-(2-méthoxy-phényl)-pipérazinyl-1]-propanol-2.

4. Le 1-[4-(1,2,4-triazolyl-1)-phénoxy]-3-[4-(4-fluorophényl-pipérazinyl-1]-propanol-2.

5. Le 1-[4-(imidazolyl-1)-phénoxy]-3-[4-(4-fluorophényl)-pipérazinyl-1]-propanol-2.

6. Le 1-[2-(pyrazolyl-3)-phénoxy]-3-[4-(2-méthoxyphényl)-pipérazinyl-1]-propanol-2.

7. Le 1-[3-(pyrazolyl-3)-phénoxy]-3-[4-(2-méthoxyphényl)-pipérazinyl-1]-propanol-2.

8. Procédé de préparation de composés de la formule générale I selon la revendication 1, caractérisé en ce que l'on fait réagir un composé de la formule générale II

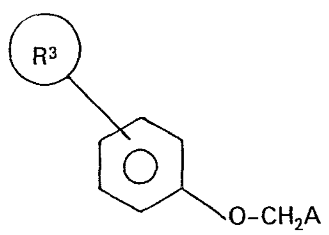

II,

dans laquelle $R^3$ peut être un groupe triazol-1-yle, imidazol-1-yle, pyrazol-3-yle ou isoxazol-3-yle, le noyau hétérocyclique pouvant être substitué par un radical alkyle en $C_1$ à $C_4$, et A désigne un groupe

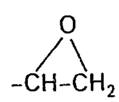

ou

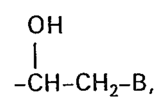

dans lequel B représente un groupe clivable nucléofuge, d'une manière connue en soi, utilement dans un solvant et éventuellement en présence d'un agent fixant les acides, avec une phénylpipérazine de la formule générale III

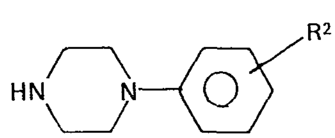

III

dans laquelle $R^2$ possède les significations définies pour la formule I, le composé obtenu pouvant le cas échéant être transformé en un sel d'addition d'un acide physiologiquement acceptable.

9. Procédé de préparation de composés de la

formule générale I selon la revendication 1, caractérisé en ce que l'on fait réagir un dérivé du 1-(dialkyl-amino-1-oxo-propène)-phénoxy-phényl-pipérazinyl-1-propanol-2 de la formule générale VI

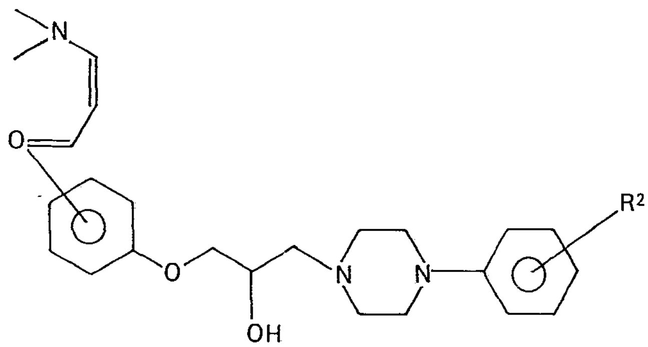

VI

de manière connue en soi, utilement en présence d'un solvant polaire et éventuellement d'une base, entre 50 et 120°C, avec de la guanidine, de l'hydroxyl-amine ou de l'hydrate d'hydrazine, puis l'on transforme éventuellement le composé obtenu, de la formule générale I, dans laquelle

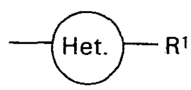

est un groupe 2-amino-pyrimidinyle-4, isoxazolyle-3 ou pyrazolyle-3, en un sel d'addition d'un acide physiologiquement acceptable.

10. Composition thérapeutique, contenant un composé de la formule I selon la revendication 1 ou un sel d'addition d'un acide physiologiquement acceptable d'un tel composé en tant que principe actif, ainsi que des véhicules et diluants usuels.